# EUROPEAN PATENT SPECIFICATION

(11) **EP 2 852 291 B1**
(45) Date of publication and mention of the grant of the patent: **06.01.2021**
(21) Application number: 13725189.8
(22) Date of filing: 14.05.2013
(51) Int. Cl.: A23D 7/00, A23D 7/06, A61K 31/115, A61K 31/202

(54) **EMULSION COMPRISING POLYUNSATURATED FATTY ACIDS.**
EMULSION MIT MEHRFACH UNGESÄTTIGTEN FETTSÄUREN
ÉMULSION COMPRENANT DES ACIDES GRAS POLYUNSATURÉS

(30) Priority: 21.05.2012 GB 201208910
(43) Date of publication of application: 01.04.2015
(73) Proprietor: Croda International PLC, Goole, East Yorkshire DN14 9AA (GB)
(72) Inventor: MELLOR, Stephen Farrall, Wetley Rocks Staffordshire ST9 0AA (GB); WEATHERHEAD, Gregory Charles, North Yorkshire HG1 5HY (GB); HUMPHREY, James Richard, Hull Yorkshire HU5 3NF (GB)
(74) Representative: Karfopoulos, Alexis Theo
(86) International application number: PCT/GB2013/051232
(87) International publication number: WO 2013/175174

(56) References cited:
- WO-A1-93/11675
- US-A- 4 913 921
- US-A1- 2010 104 730
- US-A1- 2012 040 934

## Description

### Field of Invention

The present invention relates to an emulsion comprising polyunsaturated fatty acids, to the use thereof as a dietary supplement, and in particular in packaged form.

### Background

Polyunsaturated fatty acids, particularly long chain polyunsaturated fatty acids, including omega-3, omega-6 and omega-9 fatty acids, are vital to everyday life and function. In particular, long chain omega-3 fatty acids (e.g. eicosapentaenoic acid (EPA) and docosahexanoic acid (DHA)) are known to enhance cognitive function and maintain cardiovascular health, amongst other health benefits. Recent studies have also indicated that omega-3 fatty acids can be effective in reducing the risk of coronary heart disease. Omega-3 fatty acids are not produced by the human body, but are found in natural sources such as in the oil of certain plants and animals, particularly fish, seeds, and algae. Microbes and plants have also been genetically modified to include genes that produce various polyunsaturated fatty acids in an attempt to reduce the costs associated with their production. WO93/11675 A1 describes a dietary product based on polyunsaturated fatty acids of the omega-3, omega-6 and omega-9 types and their manufacturing method, including a stabilising step comprising sparging an inert gas into the edible oil, thus resulting in a reduced oxygen content, and the addition of antioxidants.

The most widely available dietary source of EPA and DHA is cold water oily fish, such as salmon, herring, mackerel, anchovies, and sardines. Other oily fish, such as tuna, also contain omega-3 fatty acids, but in somewhat lesser amounts. Although fish is a dietary source of omega-3 fatty acids, fish do not synthesise omega-3 fatty acids, as they obtain them from algae, particularly microalgae, or plankton in their diets.

Since omega-3 fatty acids are not synthesised by the human body, they must be obtained through food or dietary supplement. Dietary supplements containing omega-3 fatty acids are normally packaged in capsules or as an emulsion in sachets or bottles (for dispensing by spoon). Food companies have recently launched a wide range of foods fortified with omega-3 fatty acids such as bread, mayonnaise, pizza, yogurt, fruit juice, children's pasta, milk, eggs, popcorn, confectionary, and infant formula.

Many polyunsaturated fatty acids are sensitive to oxidation, can have an unpleasant taste and be difficult to formulate in a stable form. There is a need for a composition, particularly in the form of an emulsion, containing polyunsaturated fatty acids at high concentration with improved stability and/or organoleptic properties, which can be used as a dietary supplement and/or be easily incorporated into food products.

### Summary of the Invention

We have now surprisingly discovered an emulsion which overcomes or significantly reduces at least one of the aforementioned problems.

Accordingly, the present invention provides an emulsion comprising
(i) 65 to 74% by weight of an aqueous phase, and
(ii) 26 to 35% by weight of an oil phase comprising;
   (a) 65 to 75% by weight of capric/caprylic triglyceride; and
   (b) 20 to 35% by weight of at least one polyunsaturated fatty acid and/or derivative thereof;
(iii) less than 4% by weight of at least one emulsifier, wherein the emulsifier is selected from an alkoxylated sorbitan ester; and
(iv) less than 7% by weight of the aqueous phase of at least one stabiliser selected from acacia gum and xanthan gum.

The invention further provides a dietary supplement comprising, consisting essentially of, or consisting of, an emulsion comprising
(i) 65 to 74% by weight of an aqueous phase, and
(ii) 26 to 35% by weight of an oil phase comprising;
   (a) 65 to 75% by weight of capric/caprylic triglyceride; and
   (b) 20 to 35% by weight of at least one polyunsaturated fatty acid and/or derivative thereof;
(iii) less than 4% by weight of at least one emulsifier, wherein the emulsifier is selected from an alkoxylated sorbitan ester; and
(iv) less than 7% by weight of the aqueous phase of at least one stabiliser selected from acacia gum and xanthan gum,
packaged in a container, where said container is a sachet or 'stick shot'.

The invention yet further provides the use of an emulsion comprising
(i) 65 to 74% by weight of an aqueous phase, and
(ii) 26 to 35% by weight of an oil phase comprising;
   (a) 65 to 75% by weight of capric/caprylic triglyceride; and
   (b) 20 to 35% by weight of at least one polyunsaturated fatty acid and/or derivative thereof;
(iii) less than 4% by weight of at least one emulsifier, wherein the emulsifier is selected from an alkoxylated sorbitan ester; and
(iv) less than 7% by weight of the aqueous phase of at least one stabiliser selected from acacia gum and xanthan gum,
as a dietary supplement.

The emulsion according to the present invention may be in the form of an oil-in-water or a water-in-oil emulsion. The emulsion is preferably an oil-in-water emulsion.

The concentration of the aqueous phase in the emulsion is 65 to 74%, and especially 68 to 71% by weight based on the total weight of the emulsion.

The concentration of the oil phase in the emulsion is 26 to 35%, and especially 29 to 32% by weight based on the total weight of the emulsion.

The polyunsaturated fatty acids present in the emulsion may be omega-3, omega-6 and/or omega-9 fatty acids and/or derivatives thereof. The polyunsaturated fatty acid derivatives include salts, e.g. alkali metal salts, alkyl esters (e.g. methyl, ethyl esters), glyceride esters (e.g. mono-, di-, and triglycerides), phospholipids (e.g. in krill oil) and sterol esters (e.g. phytosterol esters). Preferred polyunsaturated fatty acid derivatives are glyceride esters and/or ethyl esters, and particularly triglyceride esters.

Specific examples of omega-3 polyunsaturated fatty acids include hexadecatrienoic acid (HTA), alpha-linolenic acid (ALA), stearidonic acid (SDA), eicosatrienoic acid (ETE), eicosatetraenoic acid (ETA), eicosapentaenoic acid (EPA), heneicosapentaenoic acid (HPA), docosahexaenoic acid (DHA), docosapentaenoic acid (DPA), tetracosapentaenoic acid, and tetracosahexaenoic acid (nisinic acid).

Specific examples of omega-6 polyunsaturated fatty acids include linoleic acid, gamma-linolenic acid (GLA), eicosadienoic acid, dihomo-gamma-linolenic acid (DGLA), arachidonic acid (AA), docosadienoic acid, adrenic acid, docosapentaenoic acid, tetracosatetraenoic acid, and tetracosapentaenoic acid.

Omega-3 fatty acids are preferred, and particularly EPA and/or DHA.

The polyunsaturated fatty acids and/or derivatives thereof can be obtained from marine oils (e.g. fish oil, seal oil, krill oil); microbial oils (naturally occurring microbes or modified by mutagenesis or genetic engineering) such as algae oil (e.g. microalgae oil) or fungal oil; seed oils, e.g. linseed oil, perilla oil; and plant oils (naturally occurring plants or genetically modified plants).

Marine, particularly fish oils are preferred. The fish oil may be a crude, semi-refined (alkali-refined), and preferably a refined or concentrated fish oil. Suitable fish oils include Atlantic fish oil, Pacific fish oil, Mediterranean fish oil, light pressed fish oil, alkaline treated fish oil, heat treated fish oil, light and heavy brown fish oil, bonito oil, pilchard oil, tilapia oil, tuna oil, sea bass oil, halibut oil, spearfish oil, barracuda oil, cod oil, menhaden oil, sardine oil, anchovy oil, capelin oil, Atlantic cod oil, Atlantic herring oil, Atlantic mackerel oil, Atlantic menhaden oil, salmonid oil, tuna oil, and/or shark oil. Preferred fish oils are sardine, anchovy and/or tuna oil.

The polyunsaturated fatty acids and/or derivatives thereof are preferably incorporated into the oil phase of an emulsion according to the invention in the form of an oil, i.e. a polyunsaturated fatty acid and/or derivatives thereof containing oil (PUFA oil). Any one or more of the above described PUFA oils may be used. The PUFA oil is preferably a marine oil, more preferably a fish oil, and particularly a concentrated sardine, anchovy and/or tuna oil.

The concentration of the PUFA oil in the oil phase of the emulsion is suitably in the range from particularly 25 to 35%, and especially 30 to 32% by weight based on the total weight of the oil phase.

The concentration of the polyunsaturated fatty acids and/or derivatives thereof, preferably omega 3 fatty acids (both expressed as free fatty acid concentration), present in the PUFA oil is suitably greater than 35%, preferably in the range from 45 to 80%, more preferably 50 to 70%, particularly 55 to 65%, and especially 57 to 63% by weight based on the total weight of the PUFA oil.

The combined concentration of EPA and DHA (expressed as free fatty acid concentration), present in the PUFA oil is suitably greater than 30%, preferably in the range from 40 to 75%, more preferably 45 to 65%, particularly 47 to 60%, and especially 48 to 54% by weight based on the total weight of the PUFA oil.

In one embodiment, the concentration of EPA (expressed as free fatty acid concentration), present in the PUFA oil is suitably greater than 5%, preferably in the range from 15 to 50%, more preferably 20 to 40%, particularly 25 to 36%, and especially 28 to 33% by weight based on the total weight of the PUFA oil.

In another embodiment, the concentration of DHA (expressed as free fatty acid concentration), present in the PUFA oil is suitably greater than 10%, preferably in the range from 13 to 65%, more preferably 16 to 40%, particularly 18 to 30%, and especially 19 to 22% by weight based on the total weight of the PUFA oil.

The concentration of the polyunsaturated fatty acids and/or derivatives thereof, preferably omega 3 fatty acids (both expressed as free fatty acid concentration), present in the oil phase of the emulsion is suitably at least 10%, preferably in the range from 14 to 27%, more preferably 16 to 24%, particularly 18 to 22%, and especially 19 to 21% by weight based on the total weight of the oil phase.

The combined concentration of EPA and DHA (expressed as free fatty acid concentration), present in the oil phase is suitably at least 9%, preferably in the range from 12 to 25%, more preferably 14 to 22%, particularly 15 to 20%, and especially 16 to 18% by weight based on the total weight of the oil phase.

In one embodiment, the concentration of EPA (expressed as free fatty acid concentration), present in the oil phase is suitably greater than 2%, preferably in the range from 3 to 17%, more preferably 6 to 13%, particularly 8 to 12%, and especially 9 to 11% by weight based on the total weight of the oil phase.

In another embodiment, the concentration of DHA (expressed as free fatty acid concentration), present in the oil phase is suitably greater than 3%, preferably in the range from 4 to 20%, more preferably 5 to 13%, particularly 6 to 10%, and especially 7 to 8% by weight based on the total weight of the oil phase.

In one preferred embodiment, the PUFA oil suitably comprises less than 25%, preferably less than 20%, more preferably in the range from 0.5 to 15%, particularly 1 to 10%, and especially 2 to 5% by weight based on the total weight of the PUFA oil of saturated fatty acids and/or derivatives thereof (expressed as free fatty acid concentration) comprising 10 to 24, preferably 12 to 20, more preferably 14 to 18, particularly 14 to 16, and especially 16 carbon atoms in the fatty acid.

The diluent oil present in the oil phase of the emulsion of the invention is caprylic/capric triglyceride oil.

The concentration of the diluent oil in the oil phase of the emulsion is 65 to 75%, and especially 68 to 70% by weight based on the total weight of the oil phase.

In one embodiment, the diluent oil comprises substantially no hydrocarbyl, particularly alkyl, groups comprising more than 18, preferably more than 16, more preferably more than 14, particularly more than 12, and especially more than 10 carbon atoms. By "substantially no" is meant less than 3%, preferably less than 2%, particularly less than 1%, and especially less than 0.1% by weight based on the total weight of hydrocarbyl, preferably alkyl, groups defined above present in the diluent oil.

In another embodiment, the diluent oil comprises a low concentration of polyunsaturated fatty acids and/or derivatives thereof, particularly omega 3 fatty acids (both expressed as free fatty acid concentration), suitably less than 10%, preferably less than 5%, more preferably less than 3%, particularly less than 1%, and especially less than 0.1% by weight based on the total weight of the diluent oil.

In one preferred embodiment, the oil phase suitably comprises less than 12%, preferably less than 8%, more preferably less than 5%, particularly less than 3%, and especially less than 2% by weight based on the total weight of the oil phase of saturated fatty acids and/or derivatives thereof comprising 12 to 24, preferably 14 to 22, more preferably 16 to 20, particularly 18 to 20, and especially 18 carbon atoms in the fatty acid.

In addition to water, the aqueous phase may also comprise up to 20%, preferably in the range from 1 to 15%, more preferably 3 to 10%, and especially 5 to 7% by weight, based on the total weight of the aqueous phase, of a co-solvent. Suitable co-solvents include polar solvents such as propylene glycol, glycerine and/or C1 to C6 alcohols such as those selected from the group consisting of methanol, ethanol, propanol, isopropanol, butanol, isobutanol, tert-butanol, n-pentanol, iso-pentanol, n-hexanol, isohexanol, glycol, glycerol, 1,2-pentanediol, 1,5-pentanediol, 1,2-hexanediol and 1,6-hexanediol. Glycerine is a particularly preferred co-solvent.

The emulsion comprises less than 4%, preferably less than 2.5%, more preferably less than 1.5%, particularly less than 1%, and especially less than 0.6% by weight based on the total weight of the emulsion of an emulsifier of sorbitan fatty acid esters.

The emulsion according to the present invention comprises an emulsifier and/or a stabiliser. The emulsifier may be added to the emulsion to assist in the formation of the emulsion, for example by reducing the surface tension on the surface of the droplets of the dispersed, preferably oil, phase and assisting in the dispersion of the dispersed phase in the continuous, preferably aqueous, phase. Stabilisers may assist in stabilising the emulsion by preventing coalescence, flocculation, or creaming of the dispersed phase, such as by creating a repulsive interaction between the droplets of the dispersed phase, for example, by creating an ionic charge on the droplet surface which is repelled by the like charge on the surfaces of the other droplets.

The term alkoxylate emulsifier is used to refer to materials in which a hydrophobe, usually a hydrocarbyl group, is connected through the residue of a linking group having a reactive hydrogen atom to an oligomeric or polymeric chain of alkylene oxide residues. The hydrocarbyl group is typically a chain, commonly an alkyl chain, containing from 8 to 24, particularly 12 to 22, and usually 14 to 20 carbon atoms. The linking group can be an oxygen atom (hydroxyl group residue); a carboxyl group (fatty acid or ester residue); an amino group (amine group residue); or a carboxyamido (carboxylic amide residue). The alkylene oxide residues are typically residues of ethylene oxide (C₂H₄O) or propylene oxide (C₃H₆O) or combinations of ethylene and propylene oxide residues. When combinations are used the proportion of ethylene oxide residues will preferably be at least about 50 mole% and more preferably at least 75 mole%, the remainder being propylene oxide residues. In one particularly preferred embodiment, substantially all of the residues are ethylene oxide residues. The number of alkylene residues in the emulsifier molecule is preferably in the range from 2 to 200, more preferably 5 to 100, and especially 10 to 40.

One preferred class of alkoxylate emulsifier is the polysorbates. Polysorbates are alkoxylated, preferably ethoxylated, derivatives of sorbitan esters (sorbitan esterified with fatty acids). The polysorbate may be selected from the group consisting of polysorbate 20 (Tween™ 20 (ex Croda (polyoxyethylene (20) sorbitan monolaurate)), polysorbate 40 (Tween™ 40 (ex Croda (polyoxyethylene (20) sorbitan monopalmitate)), polysorbate 60 (Tween™ 60 (ex Croda (polyoxyethylene (20) sorbitan monostearate)), and polysorbate 80 (Tween™ 80 (ex Croda (polyoxyethylene (20) sorbitan monooleate)). Polysorbate 80 is particularly preferred. The number following the polyoxyethylene part refers to the total number of ethylene oxide groups present in the molecule. The number following the polysorbate part is related to the type of fatty acid associated with the polyoxyethylene sorbitan part of the molecule. Monolaurate is indicated by 20, monopalmitate by 40, monostearate by 60 and monooleate by 80.

Non-alkoxylate emulsifiers may also be used such as a fatty acid ester, ether, hemi-acetal or acetal of a polyhydroxylic compound, or a fatty acid amide which is N-substituted with the residue of a polyhydroxylic compound, especially a saccharide fatty acid ester, and a polysaccharide stabiliser.

Useful esters of polyhydroxylic compounds include saccharide esters particularly monoesters of fatty acids and a sugar, especially sucrose, fructose and/or glucose. Commercially available sugar esters are usually mixtures containing mono-ester, higher esters and sometimes free starting material (sugar). Sucrose esters are particularly useful. Such sugar esters are relatively hydrophilic emulsifiers and less hydrophilic variants can be used in which hydroxyl groups (usually only one) on the saccharide residue are etherified (or acetalated) typically with a C1 to C4 alkyl group e.g. a methyl group.

Other esters of polyhydroxylic compounds include esters of fatty acids, particularly fatty acids having from 8 to 24, usually 12 to 22, more usually 16 to 20 carbon atoms, and polyols particularly glycerol, or a polyglycerol, or an anhydro-saccharide such as sorbitan. Examples include glycerol mono-laurate, triglycerol mono-stearate and among relatively more hydrophobic emulsifiers glycerol mono-stearate and sorbitan mono-oleate, stearate or laurate.

Other ester emulsifiers include fatty acid esters of hydroxycarboxylic acids, in particular the products of trans esterification between fatty glycerides, especially mono-and di-glycerides, and polyhydroxy-carboxylic acids. These products are usually described as esters, but are typically mixtures of the starting materials and the trans-esterification products, particularly where the fatty acid residues are esterified to hydroxyl groups on the hydroxycarboxylic acids. In these products, the fatty acid typically has from 8 to 24, usually 12 to 22, more usually 16 to 20 carbon atoms and the hydroxycarboxylic acid is desirably citric acid.

Another type of emulsifier derived from sugars are saccharide hydrocarbyl ethers, hemiacetals or acetals, commonly known as hydrocarbyl, particularly alkyl, polysaccharides (more properly oligo saccharides). A further emulsifier type is of N-substituted fatty acid amides in which the N-substituent is the residue of a polyhydroxylic compound, which is commonly a saccharide residue such as a glucosyl group.

The emulsifier may be acacia gum. Acacia gum is a mixture of glycoproteins and polysaccharides and may be obtained from the sap of two species of the acacia tree; Acacia senegal and Acacia seyal. Advantageously, the acacia gum may act as both an emulsifier and a stabiliser which may improve the stability of the emulsion or reduce the need for a separate stabiliser component in the emulsion. The emulsion may comprise up to 5wt% of acacia gum. Preferably the emulsion comprises 0.5 to 5wt% acacia gum, more preferably 1 to 3wt% acacia gum.

The concentration of emulsifier present in the aqueous phase of the emulsion is suitably less than 5%, preferably in the range from 0.005 to 1%, more preferably 0.01 to 0.5%, particularly 0.05 to 0.3%, and especially 0.1 to 0.15% by weight based on the total weight of the aqueous phase. The concentration of emulsifier present in the emulsion is suitably less than 4%, preferably in the range from 0.001 to 0.7%, more preferably 0.005 to 0.4%, particularly 0.01 to 0.2%, and especially 0.05 to 0.1% by weight based on the total weight of the emulsion.

One surprising feature of the present invention is that stable emulsions, as described herein, can be produced at such low emulsifier concentrations.

Suitable stabilizers include xanthan gum. The stabiliser may also comprise a mixture of acacia gum and xanthan gum. Thixogum is a mixture of acacia gum and xanthan gum available commercially from Nexira, France.

Xanthan gum is a polysaccharide which includes mannose, glucose and glucuronic acid monomer units. The main polymer backbone is polyglucose with 3-unit acetylated side chains including glucose and glucuronic acid, typically present as a mixed potassium, sodium and calcium salt, and mannose residues. Xanthan can be obtained from bacterial fermentations, particularly of Xanthomannas campestris and related microorganisms.

The stabiliser normally forms part of the aqueous phase and the concentration of stabiliser present in the aqueous phase is suitably less than 10%, preferably in the range from 0.01 to 5%, more preferably 0.1 to 3%, particularly 0.5 to 1%, and especially 0.6 to 0.8% by weight based upon the total weight of the aqueous phase. The concentration of stabiliser present in the emulsion is less than 7%, preferably in the range from 0.01 to 4%, more preferably 0.1 to 2%, particularly 0.3 to 0.8%, and especially 0.4 to 0.6% by weight based upon the total weight of the emulsion. One surprising feature of the present invention is that stable emulsions, as described herein, can be produced at such low stabiliser concentrations.

In one embodiment, the stabiliser is mixed with the co-solvent, preferably glycerine, prior to adding to the water to form the aqueous phase of the emulsion.

The emulsion may also comprise a flavouring agent which preferably comprises any natural or synthetically prepared fruit or botanical flavouring agent or mixtures thereof. Suitable natural or artificial fruit flavouring agents include lemon, orange, grapefruit, strawberry, banana, pear, kiwi, grape, apple, mango, pineapple, passion fruit, raspberry, and mixtures thereof. The fruit flavouring agent may be in the form of concentrated juice, dried juice or oil, preferably oil. Citrus flavours, particularly lemon or orange are preferred, especially lemon. Suitable botanical flavours include Jamaica, marigold, chrysanthemum, tea, chamomile, ginger, valerian, yohimbe, hops, eriodictyon, ginseng, bilberry, rice, red wine, mango, peony, lemon balm, nut gall, oak chip, lavender, walnut, gentiam, luo han guo, cinnamon, angelica, aloe, agrimony, yarrow, and mixtures thereof. The botanical flavouring agent may be a concentrate or an extract, for example in the form of a liquid or dried to form a powder.

The concentration of flavouring agent in the emulsion is suitably less than 10%, preferably in the range from 0.01 to 5%, more preferably 0.1 to 3%, particularly 0.5 to 1%, and especially 0.6 to 0.8% by weight based on the total weight of the emulsion.

The emulsions may also comprise a natural or artificial sweetener or sweetening agent. Suitable sweeteners are natural sugars which may be granulated or powdered, and include sucrose, fructose, dextrose, maltose, lactose, xylitol, polyols, and mixtures thereof. In one preferred embodiment, the sweetener is xylitol.

In other embodiments, artificial sweeteners may be utilized in the emulsions. Suitable artificial sweeteners include, for example, saccharin, cyclamates, sucralose, acesulfam-K, L-aspartyl-L-phenylalanine lower alkyl ester sweeteners (e.g. aspartame), L-asparty 1-D - alanine amides, L-aspartyl-D-serine amides, L-aspartyl-L-1-hydroxymethylaikaneamides, L-aspartyl-1-hydroxyethyalkaneamides, L-aspartyl-D-phenylglycine esters and amides. Mixtures of sugars and artificial sweeteners may also be used.

The concentration of the sweetener, preferably natural sweetener, in the emulsion is suitably less than 30%, preferably in the range from 1 to 25%, more preferably 5 to 20%, particularly 10 to 18%, and especially 15 to 17% by weight based upon the total weight of the emulsion.

The emulsions according to the present invention may also contain an antioxidant. The antioxidant may be present in the aqueous and/or the oil phase. Suitable examples of antioxidants include a phenolic compound, a plant extract, or a sulphur-containing compound. The antioxidant may be ascorbic acid or a salt thereof, vitamin E, CoQIO, tocopherols, lipid soluble derivatives of more polar antioxidants such as ascobyl fatty acid esters (e.g. ascobyl palmitate), plant extracts (e.g. rosemary, sage and oregano oils, green tea extract), algal extracts, and synthetic antioxidants (e.g., butylated hydroxytoluene (BHT), tert-butylhydroquinone (TBHQ), butylated hydroxyanisole (BHA), ethoxyquin, alkyl gallates, hydroquinones, tocotrienols), and combinations thereof.

The emulsion may also contain additional functionally active ingredients such as colouring agents (e.g. β-carotene, lutein, caramel colouring, lycopene, turmeric, and tartazine), preservatives and pH adjusting agents (e.g. citric acid, lactic acid, malic acid, fumaric acid, tartaric acid, potassium sorbate, sodium propionate, sodium nitrate, sodium nitrite, and disodium EDTA).

The emulsions of the invention may be made by conventional emulsification and mixing methods. For example, the surfactant may be added to (i) the oil phase, which is then added to the aqueous phase, or (ii) both the combined oil and water phases, or (iii) the water phase, which is then added to the oil phase. Method (iii) is preferred. In all of these methods, the resulting mixture can then be emulsified using standard techniques. It is preferred to either heat the aqueous and oil phases usually above about 60°C, e.g. to about 80 to 85°C, or to subject the aqueous phase to high intensity mixing at lower, e.g. about ambient, temperature.

The emulsions according to the present invention are suitably stable, measured as described herein, preferably for greater than one month, more preferably greater than two months, particularly greater than three months, and especially greater than four months at 5°C, and/or at ambient temperature (23 °C), and/or at 43 °C. The stability at even higher temperatures can also be important, and therefore the dispersions according to the invention are stable, measured as described herein, suitably for greater than one week, preferably greater than two weeks, more preferably greater than 3 weeks, particularly greater than one month, and especially greater than two months at 50 °C. The emulsion is stable if no separation of the components or phases or creaming occurs.

The emulsions may be packaged in a sachet or 'stick shot', preferably in a laminate 'stick shot'. The emulsion is suitably packaged in 5 to 10 ml servings. The emulsion is preferably packed under a low oxygen atmosphere through gas flushing the packaging with a gas such as nitrogen. The emulsion may also be packaged into bottles for dispensing with a spoon. The emulsion is suitable for direct consumption as a dietary supplement, providing the individual with their recommended daily dietary intake of omega-3 fatty acids in a single 5ml serving based on the European Food Safety Authority (EFSA) ruling.

The emulsion is also suitable for incorporation into beverages (e.g. fruit juices), dairy products (e.g. yogurts), baby foods, and a wide range of processed meats, baked goods (e.g. bread), and cereals.

The invention is illustrated by the following non-limiting examples.

### Examples

### Example 1

| Ingredient | Quantity (w/w%) |
|---|---|
| Demineralised Water | 48 |
| Citric Acid | 0.2 |
| Xylitol | 16 |
| Glycerine | 4.3 |
| Lemon Oil | 0.7 |
| Guar gum | 0.25 |
| Xanthan Gum | 0.25 |
| Potassium Sorbate | 0.1 |
| β-Carotene | 0.003 |
| Tween™ 80 (ex Croda) | 0.1 |
| Capric/Caprylic Triglyceride | 20.5 |
| PUFA Oil* | 9.5 |

| | |
|---|---|
| * contains 80 mg/g EPA, 510 mg/g DHA (expressed as free fatty acid) | |

Xylitol, citric acid and potassium sorbate were mixed with water by gentle mixing in a vessel. Glycerine, xanthan gum and guar gum were mixed separately by gentle mixing until homogenous, added to the other ingredients and mixed using a high shear mixer for 5 minutes at 6,500 rpm. Capric/caprylic triglyceride, PUFA oil, lemon oil, β-Carotene and Tween™ 80 were then added and mixed gently until homogenous. The resulting mixture was homogenised (at 215 bar) and heat treated at a temperature of 95 °C for 45 seconds. The resulting emulsion was packaged into sterile containers.

The stability of the emulsion was tested and the emulsion was stable for at least four months at 5°C, at ambient temperature (23 °C), and at 43 °C.

The emulsion had a pleasant mouthfeel and taste, with no off odour or fishy odour.

### Example 2

| Ingredient | Quantity (w/w%) |
|---|---|
| Demineralised Water | 47 |
| Citric Acid | 0.2 |
| Xylitol | 16 |
| Glycerine | 4 |
| Lemon Oil | 0.7 |
| Acacia gum | 2 |
| Thixogum** | 0.5 |
| Potassium Sorbate | 0.1 |
| β-Carotene | 0.003 |
| Capric/Caprylic Triglyceride | 20 |
| PUFA Oil* | 9.5 |

| | |
|---|---|
| * contains 80 mg/g EPA, 510 mg/g DHA (expressed as free fatty acid) ** Thixogum is a mixture of acacia gum and xanthan gum in equal proportions by weight available ex. Nexira, France | |

Xylitol, citric acid, acacia gum, and potassium sorbate were mixed with water by gentle mixing in a vessel. Glycerine and thixogum were mixed separately by gentle mixing until homogenous, added to the other ingredients and mixed using a high shear mixer for 5 minutes at 6,500 rpm. Capric/caprylic triglyceride, PUFA oil, lemon oil and β-Carotene were then added and mixed gently until homogenous. The resulting mixture was homogenised (at 215 bar) and heat treated at a temperature of 95 °C for 45 seconds. The resulting emulsion was packaged into sterile containers.

The stability of the emulsion was tested and the emulsion was stable for at least four months at 5°C, at ambient temperature (23 °C), and at 43 °C.

The emulsion had a pleasant mouthfeel and taste, with no off odour or fishy odour.

## Claims

1. An emulsion comprising;
(i) 65 to 74% by weight of an aqueous phase, and
(ii) 26 to 35%
by weight of an oil phase comprising;
(a) 65 to 75% by weight of capric/caprylic triglyceride; and
(b) 20 to 35% by weight of at least one polyunsaturated fatty acid and/or derivative thereof;
(iii) less than 4% by weight of at least one emulsifier, wherein the emulsifier is selected from an alkoxylated sorbitan ester; and
(iv) less than 7% by weight of the aqueous phase of at least one stabiliser selected from acacia gum and xanthan gum.

2. The emulsion according to claim 1 wherein the oil phase comprises 25 to 35% by weight of a PUFA oil.

3. The emulsion according to claim 2 wherein the PUFA oil comprises greater than 35% by weight of omega 3 fatty acids and/or derivatives thereof.

4. The emulsion according to claim 2 wherein the PUFA oil comprises 45 to 80% by weight of polyunsaturated fatty acids.

5. The emulsion according claims 2 wherein the PUFA oil comprises greater than 30% by weight of EPA and DHA.

6. The emulsion according to any one of the preceding claims wherein the oil phase comprises 12 to 25% by weight of EPA and DHA.

7. The emulsion according to any one of claims 2 to 6 wherein the PUFA oil comprises less than 20% by weight of saturated fatty acids and/or derivatives thereof having 10 to 24 carbon atoms.

8. The emulsion according to any one of the preceding claims wherein the capric/caprylic triglyceride comprises substantially no hydrocarbyl groups comprising more than 16 carbon atoms.

9. The emulsion according to any one of the preceding claims wherein the stabiliser is xanthan gum.

10. The emulsion according to any one of the preceding claims comprising capric/caprylic triglyceride, concentrated fish oil, alkoxylated sorbitan ester, guar gum and xanthan gum, and optionally xylitol and/or lemon oil.

11. A dietary supplement comprising the emulsion defined in any one of the preceding claims packaged in a container, where said container is a sachet or 'stick shot'.

12. The use of an emulsion as defined in any one of claims 1 to 10 as a dietary supplement.

13. The use according to claim 12 wherein the emulsion is packaged in a sachet or 'stick shot'.

## Patentansprüche

1. Emulsion, umfassend:
(i) 65 bis 74 Gew.-% an einer wässrigen Phase und
(ii) 26 bis 35 Gew.-% an einer Ölphase umfassend:
(a) 65 bis 75 Gew.-% Caprin-/Caprylsäuretriglycerid; und
(b) 20 bis 35 Gew.-% an wenigstens einer mehrfach ungesättigten Fettsäure und/oder einem Derivat davon;
(iii) weniger als 4 Gew.-% an wenigstens einem Emulgator, wobei der Emulgator ausgewählt ist aus einem alkoxylierten Sorbitanester; und
(iv) weniger als 7 Gew.-% der wässrigen Phase an wenigstens einem Stabilisator ausgewählt aus Gummiarabicum und Xanthangummi.

2. Emulsion gemäß Anspruch 1, wobei die Ölphase 25 bis 35 Gew.-% an einem PUFA-Öl umfasst.

3. Emulsion gemäß Anspruch 2, wobei das PUFA-Öl mehr als 35 Gew.-% Omega-3-Fettsäuren und/oder Derivate davon umfasst.

4. Emulsion gemäß Anspruch 2, wobei das PUFA-Öl 45 bis 80 Gew.-% mehrfach ungesättigte Fettsäuren umfasst.

5. Emulsion gemäß Anspruch 2, wobei das PUFA-Öl mehr als 30 Gew.-% EPA und DHA umfasst.

6. Emulsion gemäß einem der vorstehenden Ansprüche, wobei die Ölphase 12 bis 25 Gew.-% EPA und DHA umfasst.

7. Emulsion gemäß einem der Ansprüche 2 bis 6, wobei das PUFA-Öl weniger als 20 Gew.-% gesättigte Fettsäuren und/oder Derivate davon mit 10 bis 24 Kohlenstoffatomen umfasst.

8. Emulsion gemäß einem der vorstehenden Ansprüche, wobei das Caprin-/Caprylsäuretriglycerid im Wesentlichen keine Hydrocarbylgruppen umfasst, die mehr als 16 Kohlenstoffatome umfassen.

9. Emulsion gemäß einem der vorstehenden Ansprüche, wobei der Stabilisator Xanthangummi ist.

10. Emulsion gemäß einem der vorstehenden Ansprüche, umfassend Caprin-/Caprylsäuretriglycerid, konzentriertes Fischöl, alkoxylierten Sorbitanester, Guargummi und Xanthangummi und gegebenenfalls Xylit und/oder Zitronenöl.

11. Nahrungsergänzungsmittel, umfassend die Emulsion gemäß einem der vorstehenden Ansprüche in einem Behälter verpackt, wobei der Behälter ein Sachet oder "Stick Shot" ist.

12. Verwendung einer Emulsion gemäß einem der Ansprüche 1 bis 10 als Nahrungsergänzungsmittel.

13. Verwendung gemäß Anspruch 12, wobei die Emulsion in einem Sachet oder "Stick Shot" verpackt ist.

## Revendications

1. Émulsion comprenant :
(i) 65 à 74 % en poids d'une phase aqueuse, et
(ii) 26 à 35 % en poids d'une phase huileuse comprenant :
(a) 65 à 75 % en poids d'un triglycéride caprique/caprylique ; et
(b) 20 à 35 % en poids d'au moins un acide gras polyinsaturé et/ou d'un dérivé de celui-ci ;
(iii) moins de 4 % en poids d'au moins un émulsifiant, l'émulsifiant sélectionné étant un ester de sorbitane alcoxylé ; et
(iv) moins de 7 % en poids de la phase aqueuse d'au moins un stabilisant sélectionné parmi la gomme d'acacia et la gomme xanthane.

2. Émulsion selon la revendication 1, dans laquelle la phase huileuse comprend 25 à 35 % en poids d'une huile contenant des acides gras polyinsaturés.

3. Émulsion selon la revendication 2, dans laquelle l'huile contenant des acides gras polyinsaturés comprend plus de 35 % en poids d'acides gras oméga 3 et/ou de dérivés de ceux-ci.

4. Émulsion selon la revendication 2, dans laquelle l'huile contenant des acides gras polyinsaturés comprend 45 à 80 % en poids d'acides gras polyinsaturés.

5. Émulsion selon la revendication 2, dans laquelle l'huile contenant des acides gras polyinsaturés comprend plus de 30 % en poids d'EPA et de DHA.

6. Émulsion selon l'une quelconque des revendications précédentes, dans laquelle la phase huileuse comprend 12 à 25 % en poids d'EPA et de DHA.

7. Émulsion selon l'une quelconque des revendications 2 à 6, dans laquelle l'huile contenant des acides gras polyinsaturés comprend moins de 20 % en poids d'acides gras saturés et/ou de dérivés de ceux-ci comportant 10 à 24 atomes de carbone.

8. Émulsion selon l'une quelconque des revendications précédentes, dans laquelle le triglycéride caprique/caprylique ne comprend pratiquement aucun groupe hydrocarbyle comprenant plus de 16 atomes de carbone.

9. Émulsion selon l'une quelconque des revendications précédentes, dans laquelle le stabilisant est la gomme xanthane.

10. Émulsion selon l'une quelconque des revendications précédentes, comprenant un triglycéride caprique/caprylique, de l'huile de poisson concentrée, un ester de sorbitane alcoxylé, de la gomme de guar et de la gomme xanthane, et optionnellement du xylitol et/ou de l'huile de citron.

11. Complément alimentaire comprenant l'émulsion définie dans l'une quelconque des revendications précédentes emballée dans un contenant, ledit contenant étant un sachet ou un « stick ».

12. Utilisation d'une émulsion telle que définie dans l'une quelconque des revendications 1 à 10 comme complément alimentaire.

13. Utilisation selon la revendication 12, dans laquelle l'émulsion est emballée dans un sachet ou un « stick ».
